# EUROPEAN PATENT SPECIFICATION

(11) **EP 1 294 893 B1**
(45) Date of publication and mention of the grant of the patent: **15.03.2006**
(21) Application number: 01943625.2
(22) Date of filing: 22.06.2001
(51) Int. Cl.: C12N 15/51, C07K 14/02, A61K 39/29, A61P 31/20

(54) **MODIFICATION OF HEPATITIS B CORE ANTIGEN**
MODIFIZIERUNG DES HEPATITIS B KERNANTIGENS
MODIFICATION DE L'ANTIGENE CAPSIDIQUE DE L'HEPATITE B

(30) Priority: 22.06.2000 GB 0015308; 06.10.2000 GB 0024544
(43) Date of publication of application: 26.03.2003
(73) Proprietor: UCB Pharma Limited, Slough Berkshire SL1 3WE (GB)
(72) Inventor: PAGE, Mark, Welwyn Garden City Hertfordshire AL1 1BY (GB); LI, Jing-Li, Beckenham Kent BR3 4TW (GB); PUMPENS, Paul Biomedical Research and Study Centre, LV-1067 (LV); BORISOVA, Galina, Biomedical Research& Study Ctr., Riga, Latvia 1067 (LT)
(74) Representative: Campbell, Patrick John Henry
(86) International application number: PCT/GB2001/002817
(87) International publication number: WO 2001/098333

(56) References cited:
- EP-A- 0 421 635
- WO-A-00/26385
- HATTON T ET AL: "RNA AND DNA-BINDING ACTIVITIES IN HEPATITIS B VIRUS CAPSID PROTEIN A MODEL FOR THEIR ROLES IN VIRAL REPLICATION" JOURNAL OF VIROLOGY, vol. 66, no. 9, 1992, pages 5232-5241, XP001041852 ISSN: 0022-538X
- SCHOEDEL F ET AL: "HYBRID HEPATITIS B VIRUS CORE ANTIGEN AS A VACCINE CARRIER MOIETY: I. PRESENTATION OF FOREIGN EPITOPES" JOURNAL OF BIOTECHNOLOGY, ELSEVIER SCIENCE PUBLISHERS, AMSTERDAM, NL, vol. 44, no. 1/3, January 1996 (1996-01), pages 91-96, XP000891474 ISSN: 0168-1656
- BORISOVA G P ET AL: "RECOMBINANT CORE PARTICLES OF HEPATITIS B VIRUS EXPOSING FOREIGN ANTIGENIC DETERMINANTS ON THEIR SURFACE" FEBS LETTERS, ELSEVIER SCIENCE PUBLISHERS, AMSTERDAM, NL, vol. 259, no. 1, 1 December 1989 (1989-12-01), pages 121-124, XP002035980 ISSN: 0014-5793
- HUI ERIC KA-WAI ET AL: "Hepatitis B virus maturation is affected by the incorporation of core proteins having a C-terminal substitution of arginine or lysine stretches." JOURNAL OF GENERAL VIROLOGY, vol. 80, no. 10, October 1999 (1999-10), pages 2661-2671, XP002185072 ISSN: 0022-1317
- DATABASE BIOSIS [Online] BIOSCIENCES INFORMATION SERVICE, PHILADELPHIA, PA, US; 1992 ZHOU S ET AL: "CYS RESIDUES OF THE HEPATITIS B VIRUS CAPSID PROTEIN ARE NOT ESSENTIAL FOR THE ASSEMBLY OF VIRAL CORE PARTICLES BUT CAN INFLUENCE THEIR STABILITY" Database accession no. PREV199294115400 XP002185073 & JOURNAL OF VIROLOGY, vol. 66, no. 9, 1992, pages 5393-5398, ISSN: 0022-538X

## Description

The invention relates to modified forms of the core antigen of hepatitis B virus (HBV) and to prophylactic and therapeutic vaccines containing the modified antigen.

### Background to the invention

HBV remains a major healthcare problem throughout both the developed and developing world. Infection with the virus can result in an acute or chronic disease which in a proportion of cases may lead to hepatocellular carcinoma and death. The virus is double shelled, and its DNA is protected inside a protein structure called the core antigen (HBcAg). The core is surrounded by the envelope protein known as the surface or S antigen (HBsAg).

HBcAg is an unusual antigen which can be used as a delivery vehicle for specific peptides to the immune system. The antigen has been used to present T-helper, B and cytotoxic lymphocyte (CTL) epitopes from a variety of viral and bacterial pathogens, including epitopes from the surface antigen of HBV, envelope proteins from hepatitis A and antigens from hepatitis C virus. For a review see Ulrich et al (1998) Advances in Virus Research 50 141-182.

HBcAg is an excellent vehicle for the presentation of epitopes due to the molecular structure of the protein, which self-assembles into particles. Each particle is generated from either 180 or 240 copies of a monomeric polypeptide. The polypeptide has 183 or 185 amino acids (aa) depending on the subtype of HBV. The monomer, on reaching an appropriate concentration inside the host cell, forms a particle of approximately 27 nm in diameter. Structural studies have shown that amino acids within the region from residues 68 to 90 form a spiked structure on the surface of the particle which is known as the e1 loop. Two monomers joined by disulphide bonds link to form a dimer spike, the most exposed amino acid being at position 80 (at the centre of the e1 loop).

EP-A-421635 (The Wellcome Foundation Limited) describes modification of the HBV core gene to allow insertion of foreign epitopes into the e1 loop without altering the potential of the protein to from particles. Insertion at this site allows maximum exposure of the inserted epitope on the tip of each spike created by dimers of the protein. As there are approximately 180 (or 240) copies of each monomer per particle, each particle is able to present 180 (or 240) copies of the epitope of interest.

Thus, HBcAg can be used to generate hybrid particles to be used as prophylactic and therapeutic vaccines against infectious diseases. However, initial work has identified a high nucleic acid impurity profile due to the inherent nature of the core protein to bind nucleic acid. The binding of nucleic acid is known to be associated with four arginine repeats found at the C-terminus of the protein. Removal of these repeats using genetic tools has been shown to be feasible and results in the production of particles which do not encapsidate nucleic acid. However, removal of this region appears to reduce the inherent stability of the particle structure.

### Summary of the invention

In order to maintain particle stability, whilst overcoming the problem of nucleic acid impurity, the inventors have devised an alternative and novel strategy. The strategy involves generating a clone in which one or more of the arginine repeats of HBcAg is removed but in which the C-terminal cysteine is retained. The removal of the arginine repeats reduces binding of nucleic acid, whilst retention of the C-terminal cysteine allows the formation of a disulphide bond which in the native structure is important for the formation of a stable particle. The deleted repeat(s) may be replaced with sequences encoding T-helper, B or CTL epitopes from bacterial or viral pathogens, parasites, allergens or cancer associated antigens. This is made possible by insertion of a suitable cloning site in place of the deleted region.

Thus, the invention provides a protein comprising HBcAg wherein one or more of the four arginine repeats is absent and a C-terminal cysteine residue is present. An epitope from a protein other than HBcAg may be present in place of the absent arginine repeat(s). The protein may be incorporated into a pharmaceutical composition for prophylactic or therapeutic vaccination, for example against HBV.

The protein of the invention may comprise a second epitope from a protein other than HBcAg, and the second epitope may be in the e1 loop of HBcAg. By placing a T-helper epitope in the C-terminus and a B-cell epitope in the e1 loop, it is possible to enhance the response to the B-cell epitope through intrastructural T-cell help. In addition, the strategy can be used to double the number of a particular epitope on each particle, by cloning the same sequence into both the e1 loop and the C-terminal region.

### Brief description of the drawings

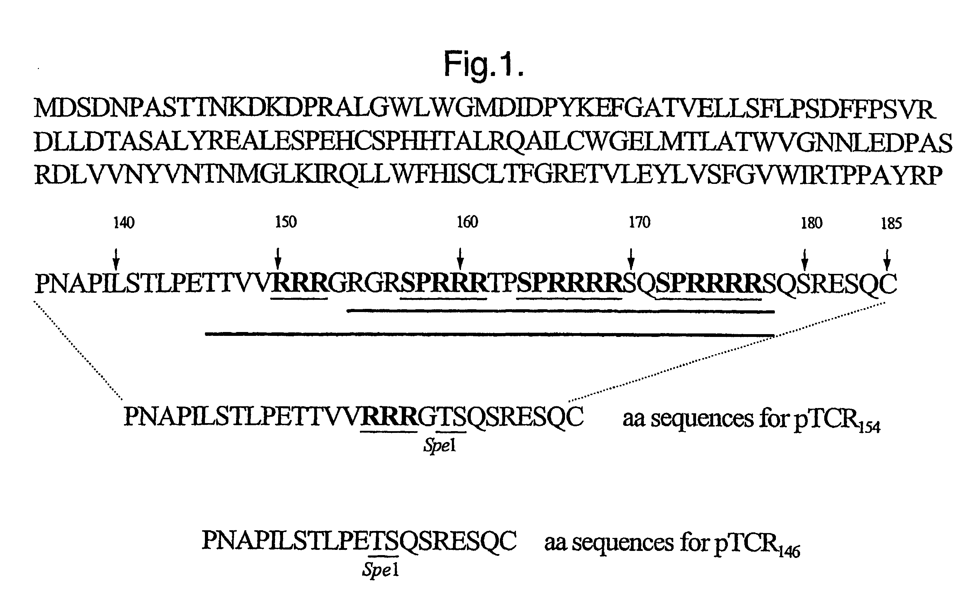

**Figure 1**: Amino acid sequence of hepatitis B core using the single letter code. The C-terminal sequence (aa135-185) is highlighted to detail the deletion strategy. The 4 arginine (R) repeats are emboldened and underlined for emphasis. Three or four arginine repeat regions are underlined from aa154-178 or aa146-178 respectively. Deletion of the underlined regions with insertion of the *SpeI* restriction site generates constructs encoded by plasmids pTCR₁₅₄ and pTCR₁₄₆ respectively. pTCR₁₅₄ retains the N-terminal arginine repeat, and pTCR₁₄₆ has all 4 arginine repeats deleted.

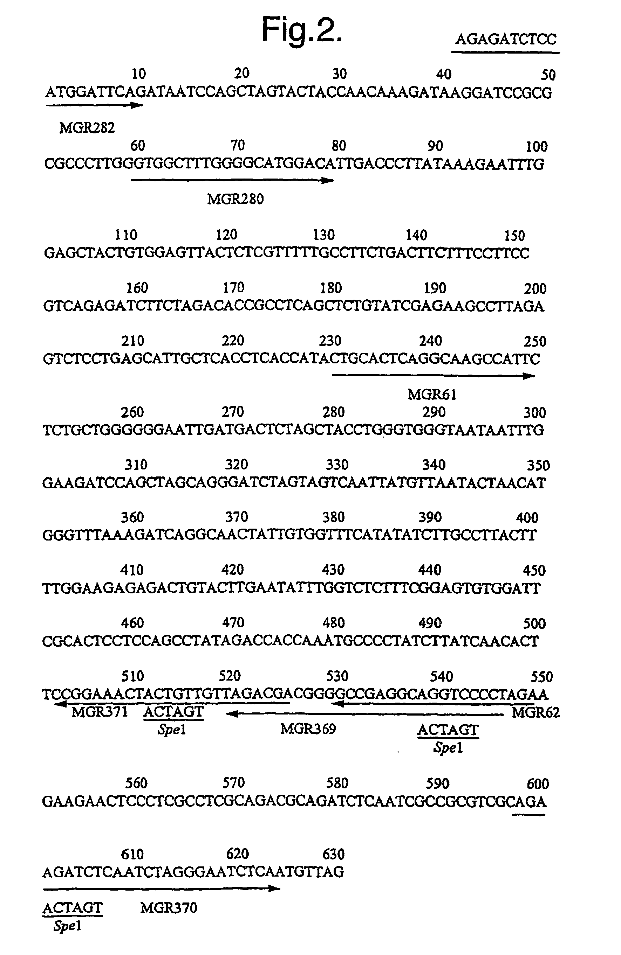

**Figure 2**: DNA sequence coding for HBcAg and location and orientation of oligonucleotide primers used for PCR. The position of the *Spe*I restriction site is given for oligos MGR371, MGR369 and MGR370 (see Table 1).

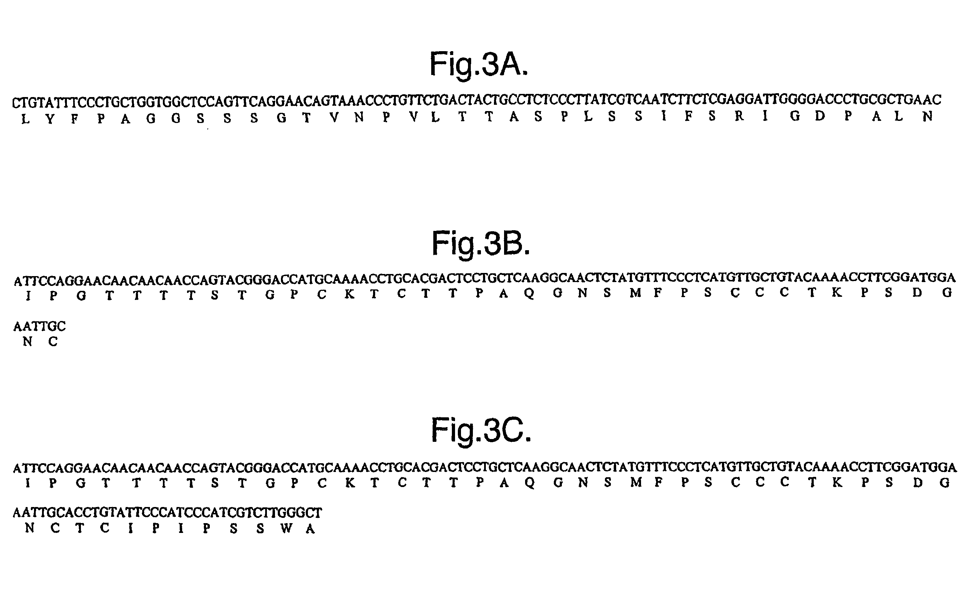

**Figure 3**: DNA and amino acid sequences of pre-S2 and S epitopes inserted into core. Figure 3A shows the sequence of aa20-55 of the pre-S2 region of the HBV ayw subtype. Figure 3B shows the sequence of aa110-147 of the S antigen of the adw subtype. Figure 3C shows the sequence of aa110-157 of the S antigen of the adw subtype.

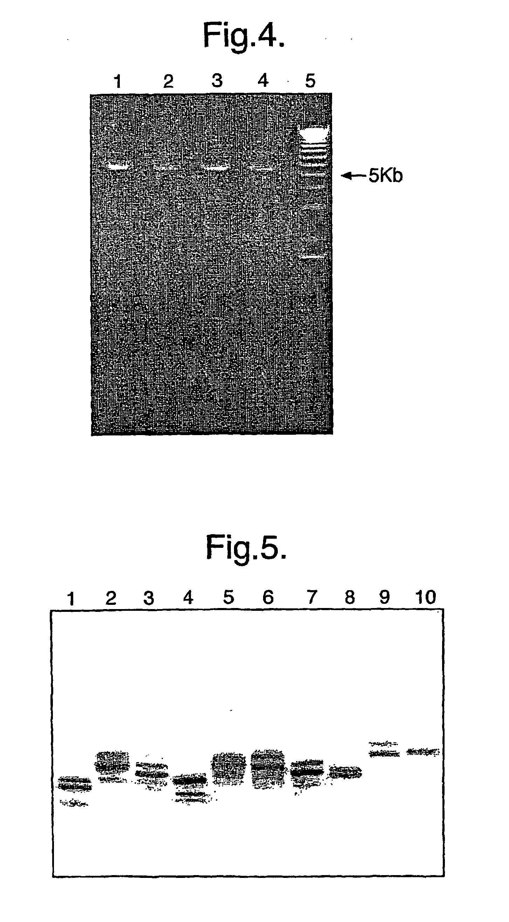

**Figure 4**: Agarose gel electroporesis of inverse PCR fragments. Lanes 1, 2, 3 and 4 = fragments for pTCR₁₄₆, pTCR₁₅₄, pTCSR₁₄₆ and pTCSR₁₅₄ respectively. Lane 5 = size markers. All fragments are of about 5kb as expected.
**Figure 5**: Immunoblot analysis of expression of core protein in lysates of *E.coli* bacteria transformed with 3' replacement plasmid constructs. All samples express an anti-core antibody reactive protein of various relative molecular weights depending on presence or absence of replacement sequences and size of replacement. Sample order:
   Lane 1 = pTCR₁₄₆ *E.coli* HB101
   Lane 2 = pTCR₁₄₆/S110-157 *E.coli* HB101
   Lane 3 = pTCR₁₄₆/S2-2 *E.coli* HB101
   Lane 4 = pTCR₁₅₄ E.*coli* HB101
   Lane 5 = pTCR₁₅₄/S110-147 *E.coli* HB101
   Lane 6 = pTCR₁₅₄/S 110-157 *E. coli* HB101
   Lane 7 = pTCR₁₅₄/S2-2 *E.coli* HB101
   Lane 8 = pTCSR₁₄₆ *E.coli* BB101
   Lane 9 = pTCSR₁₄₆/S 110-157 *E.coli* HB101
   Lane 10 = pTCSR₁₄₆/S2-2 *E.coli* HB101.

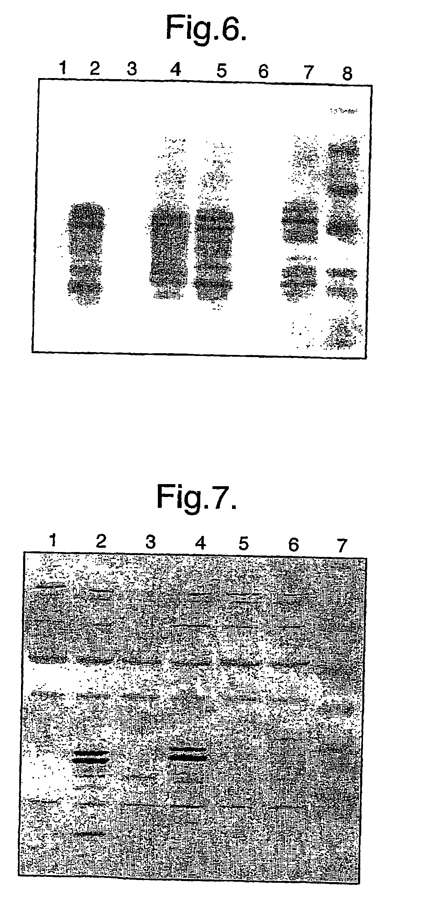

**Figure 6**: Immunoblot analysis of expression of S sequence in lysates of bacteria transformed with 3' replacement plasmid constructs. Constructs incorporating the S sequences (lanes 2, 4, 5 and 7) are anti-S antibody reactive. Sample order:
   Lane 1 = pTCR₁₄₆ *E. coli* HB 101
   Lane 2 = pTCR₁₄₆/S110-157 *E*.*coli* HB 101
   Lane 3 = pTCR₁₅₄ *E.coli* HB101
   Lane 4 = pTCR₁₅₄/S110-147 *E.coli* HB101
   Lane 5 = pTCR₁₅₄/S110-157 *E.coli* HB101
   Lane 6 = pTCSR₁₄₆ *E.coli* HB101
   Lane 7 = pTCSR₁₄₆/S 110-157 *E. coli* HB101
   Lane 8 = Pre-stain marker (Novex).
**Figure 7**: Immunoblot analysis of expression of pre-S2 sequence in lysates of bacteria transformed with 3' replacement plasmid constructs. Constructs incorporating the pre-S2 sequences (lanes 2, 4 and 6) are pre-S2 antibody reactive. Sample order:
   Lane 1 = pTCR₁₄₆ *E.coli* HB101
   Lane 2 = pTCR₁₄₆/S2-2 *E.coli* HB101
   Lane 3 =pTCR₁₅₄ *E.coli* HB101
   Lane 4 = pTCR₁₅₄/S2-2 *E.coli* HB101
   Lane 5 = pTCSR₁₄₆ *E.coli* HB101
   Lane 6 = pTCSR₁₄₆/S2-2 *E.coli* HB101
   Lane 7 = Pre-stain marker (Novex).
**Figure 8**: shows averaged anti-HBc responses in mice immunised with various constructs described in the Examples. The titers were calculated as the negative logarithms of the EC50 (effective concentration, 50%) serum dilution on the basis of sigmoidal dose-response curves.

### Detailed description of the invention

### The modifications to the HBcAg sequence

As mentioned above, HBcAg is a protein of 183 or 185 amino acids depending on the subtype ofHBV. The extra two amino acids in the 185 form of the protein are located between the first and the second arginine repeats. The sequence of a 185 amino acid form of the protein with a pre-sequence is shown in Figure 1. In Figure 1, the mature HBcAg sequence runs from the Met residue at position 25 to the Cys residue at the extreme C-terminus, with the sequence from residues 1 to 24 being the pre-sequence. The four arginine repeats are located at the following positions:

| | Position in mature 183 aa sequence | Position in mature 185 aa sequence (see Figure 1) |
|---|---|---|
| first repeat | 150-152 | 150-152 |
| second repeat | 157-159 | 159-161 |
| third repeat | 164-167 | 166-169 |
| fourth repeat | 172-175 | 174-177 |

One or more of the arginine repeats is deleted in the protein of the invention. Thus, it is possible to delete one, two, three or all four of the repeats and to delete the first repeat, the second repeat, the third repeat and/or the fourth repeat. Any combination of the four repeats may be deleted. The first repeat is primarily responsible for RNA binding and the second, third and fourth repeats are primarily responsible for DNA binding, and in a preferred embodiment the first repeat is retained and the second to fourth repeats are deleted in order to specifically reduce DNA binding.

A sequence lying between residues 145 and 182 of HBcAg is generally absent in the proteins of the invention, and preferably a sequence lying between residues 150 and 177 is absent. The deleted sequence may comprise the whole of the sequence from residue 145 to residue 182 (or from residue 150 to residue 177) or may comprise only a part of the sequence between those residues. Equally, the deleted sequence may extend on either side of those residues. As used herein, expressions such as "a sequence lying between residues x and y is absent" mean that the sequence which is absent may include residues x and y. Removal of sequence upstream of residue 145 may interfere with the particle-forming ability of the protein and is therefore generally not recommended. In 185 aa forms of HBcAg the deleted sequence may end at residue 184, and in 183 aa forms it may end at residue 182.

The C-terminal cysteine residue in the protein of the invention is typically the natural residue from the C-terminus ofHBcAg and is typically preceded by the sequence immediately upstream of the residue in HBcAg. The preceding HBcAg sequence may comprise from 1 to 7 residues, i.e. 1, 2, 3, 4, 5, 6 or 7 residues. Thus, the C-terminus of the protein of the invention may have the sequence Gln Cys, Ser Gln Cys, Glu Ser Gln Cys, Arg Glu Ser Gln Cys, Ser Arg Glu Ser Gln Cys, Gln Ser Arg Glu Ser Gln Cys or Se, Gln Ser Arg Glu Ser Gln Cys. However, the Cys residue may not be the one from HBcAg; in this case, a protein according to the invention may be constructed by truncating the HBcAg sequence and replacing the truncated sequence with another sequence including a Cys residue and optionally an epitope from a protein other than HBcAg. The Cys residue is typically located at the extreme C-terminal end of the protein of the invention but it may be a number of amino acid residues from the extreme C-terminal end. For example, it may be from 1 to 20, from 1 to 10 or from 1 to 5 residues from the C-terminus. In any event, the Cys residue must be able to form a disulphide bond.

The protein of the invention typically comprises the following elements linked in an N-terminal to C-terminal direction:
(i) an N-terminal part of HBcAg which mediates the formation of particles, for example residues 1 to 144 (or 1 to 146 or 1 to 154), and
(ii) a C-terminal part of HBcAg comprising the C-terminal cysteine;
wherein at least a part of the sequence of HBcAg from between said N-terminal part and said C-terminal part comprising one or more of the arginine repeats is absent.

Where the protein also comprises an epitope from a protein other than HBcAg in place of the absent arginine repeat(s), the protein typically comprises the following elements linked in an N- to C-terminal direction:
(i) an N-terminal part of HBcAg which mediates the formation of particles, for example residues 1 to 144 (or 1 to 146 or 1 to 154),
(ii) an epitope from a protein other than HBcAg, and
(iii) a C-terminal part of HBcAg comprising the C-terminal cysteine;
wherein at least part of the sequence of HBcAg between said N-terminal part and said C-terminal part comprising one or more of the arginine repeats is absent and is replaced by said epitope.

Where the protein comprises an epitope from a protein other than HBcAg in the e1 loop, the protein typically comprises the following elements linked in an N- to C-terminal direction:
(i) an N-terminal part of the HBcAg sequence comprising e.g. residues 1 to 67 (or 1 to 74 or 1 to 79),
(ii) an epitope from a protein other than HBcAg,
(iii) a second part of the HBcAg sequence comprising e.g. residues 91 to 144 (or 91 to 146, 91 to 154, 86 to 144, 86 to 146, 86 to 154, 80 to 144, 80 to 146 or 80 to 154); and
(iv) a third part of the HBcAg sequence comprising the C-terminal cysteine;
wherein at least a part of the sequence of HBcAg from between residue 145 (or 147 or 155) and the C-terminal cysteine comprising one or more of the arginine repeats is absent.

Where the protein of the invention comprises both a first epitope from a protein other than HBcAg in place of the absent arginine repeat(s) and a second epitope from a protein other than HBcAg in the e1 loop, the protein typically comprises the following elements linked in an N- to C-terminal direction:
(i) an N-terminal part of the HBcAg sequence comprising e.g. residues 1 to 67 (or 1 to 74 or 1 to 78);
(ii) an epitope from a protein other than HBcAg,
(iii) a second part of the HBcAg sequence comprising e.g. residues 91 to 144 (or 91 to 146, 91 to 154,86 to 144, 86 to 146, 86 to 154, 80 to 144, 80 to 146 or 80 to 154);
(iv) a further epitope from a protein other than HBcAg, and
(v) a third part of the HBcAg sequence comprising the C-terminal cysteine;
wherein at least a part of the sequence of HBcAg from between residue 145 (or 147 or 155) and the C-terminal cysteine comprising one or more of the arginine repeats is absent.

As will be apparent from the above, the inventors specifically contemplate modifying the HBcAg sequence in a number of ways, including deletion of one or more of the arginine repeats, insertion of a heterologous epitope in place of the deleted repeat(s) and insertion of a second heterologous in the e1 loop. However, further modification of the HBcAg sequence is possible. Such further modification may be by way of substitution, insertion, deletion or extension. The size of an insertion, deletion or extension may, for example, be from 1 to 200 aa, from 1 to 100 aa or from 1 to 50 aa, from 1 to 20 aa or from 1 to 6 aa in the sequence of HBcAg. Substitutions may involve a number of amino acids up to, for example, 1, 2, 5,10, 20 or 50 amino acids over the length of the HBcAg sequence. The modified protein generally retains the ability to form particles. Substitutions will generally be conservative and may be made, for example, according to the following Table, in which amino acids in the same block in the second column and preferably in the same line in the third column may be substituted for each other.

| | | |
|---|---|---|
| ALIPHATIC | Non-polar | GAP |
| | | ILV |
| | Polar-uncharged | C S T M |
| | | NQ |
| | Polar-charged | DE |
| | | KR |
| AROMATIC | | HFWY |

Each part of the HBcAg sequence in the protein of the invention preferably has at least 70% sequence identity to the corresponding sequence of a natural HBcAg protein, such as the protein having the sequence shown in SEQ ID NO: 2. More preferably, the identity is at least 80%, at least 90%, at least 98%, at least 97% or at least 99%. Methods of measuring protein sequence (and nucleic acid sequence) identity are well known in the art. For example, the UWGCG Package provides the BESTFIT programme (Devereux *et al* (1984) *Nucleic Acids Research* 12, p.387-395). Similarly, the PILEUP and BLAST algorithms can be used to line up sequences (for example as described in Altschul S. F. (1993) *J Mol. Evol.* 36:290-300 and Altschul, S. F. *et al* (1990) *J*. *Mol. Biol.* 215:403-10).

The protein of the invention may self-assemble into particles which may closely resemble the particles formed by native HBcAg. The particles may be from 20 to 40 nm in diameter, but are preferably about 27 nm in diameter (which is the size of native HBcAg particles). They contain no detectable or reduced amounts of nucleic acid (DNA and RNA) compared to particles of native HBcAg. They may contain from 160 to 260 monomers of the protein of the invention, but preferably they contain approximately 180 or approximately 240 monomers (which are the numbers of monomers in native HBcAg particles).

Determination of the particulate nature of a protein according to the invention may be carried out by size exclusion chromatography and/or electron microscopy. Determination of the DNA content of the particles may be carried out by agarose gel electrophoresis or spectrophotometry. A method adapted from Birnbaum and Nasal (1990, J. Virology 64 3319-3330) may be used. The protein may be digested with Proteinase K and the nucleic acid extracted using a commercial DNA recovery kit (*e.g*. Qiagen, QIAquick™ PCR Purification Kit). Purified DNA may be visualised using a high sensitivity DNA stain (e.g. Novex, SYBER Green I™) in a 1.5% agarose gel, following electrophoresis. The DNA product obtained following extraction may be quantified using the optical density (OD) 260nm:280nm ratio according to Sambrook *et al.* (1989, Molecular cloning - A laboratory manual, second edition, published by Cold Spring Harbor Laboratory Press), for example using a Pharmacia Biotech Ultraspec 2000™.

### The epitopes

As a general rule, epitopes inserted into the protein of the invention should not prevent the folding of HBcAg or its self-assembly into particles. In addition, for improved immunogenicity, B-cell epitopes should be displayed on the surface of the particle. T-cell epitopes do not need to be displayed on the surface of the particle for optimal presentation.

There are three preferred regions for insertion of the epitopes, namely the C-terminus in place of deleted arginine repeat(s), the e1 loop and the N-terminus. These three regions all tolerate well insertion of foreign sequences. When an epitope is placed in the e1 loop of HBcAg, it may be inserted in the sequence of amino acid residues 68 to 90, 69 to 90, 71 to 90, 75 to 85 or 78 to 83. Most preferred is to insert the epitope between residues 79 and 80 or 80 and 81. HBcAg residues from the e1 loop may be deleted in proteins of the invention, so that the inserted epitope may replace all or part of the sequence of the loop.

A heterologous epitope present in a protein of the invention may be a B-cell epitope or a T-cell epitope. In the case that an epitope is a T-cell epitope it may be a T-helper (Th) cell epitope (either a Th1 or Th2 epitope) or a cytotoxic lymphocyte (CTL) epitope.

The protein of the invention may contain more than one heterologous epitope, for example up to 2, 3, 5 or 8 heterologous epitopes, and in this case each epitope may be present in the same site or at different sites in HBcAg. In a preferred embodiment of the invention, one of the epitopes is a T-helper cell epitope and another is a B-cell or a CTL epitope. The presence of the T-helper cell epitope enhances the immune response against the B-cell or CTL epitope. Where there are two or more heterologous epitopes in the protein of the invention, they may be from the same organism or the same protein. Indeed, the epitopes may be the same; this allows a doubling or further multiplication of the number of the epitope presented on the particles.

The size of the sequence comprising an epitope inserted in the protein of the invention can vary between broad limits, but will generally be from 6 to 120 aa, for example from 6 to 80 aa or 6 to 40 aa. The epitope may be conformational or linear.

The choice of epitope depends on the disease that it is wished to vaccinate against. Typically, the epitope is from a pathogen, such as a virus, a bacterium or a protozoan, but it may also be from a cancer associated antigen or an allergen. Examples of pathogens whose epitopes may be inserted include hepatitis A virus (HAV), HBV, hepatitis C virus (HCV), influenza virus, foot-and-mouth disease virus, poliovirus, herpes simplex virus, rabies virus, feline leukemia virus, human immunodeficiency virus type 1 (HIV1), human immunodeficiency virus type 2 (HIV2), simian immunodeficiency virus (SIV), human rhinovirus, dengue virus, yellow fever virus, human papilloma virus, *Plasmodium falciparum* (a cause of malaria) and bacteria such as *Mycobacteria, Bordetella, Salmonella, Escherichia, Vibrio, Haemophilus, Neisseria, Yersinia* and *Brucella.*

Specifically, the bacterium may be *Mycobacterium tuberculosis -* the cause of tuberculosis; *Bordetella pertussis* or *Bordetella parapertussis -* causes of whooping cougl *Salmonella typhimurium* - the cause of salmonellosis in several animal species; *Salmonella typhi* - the cause of human typhoid; *Salmonella enteritidis -* a cause of food poisoning in humans; *Salmonella choleraesuis* - a cause of salmonellosis in pigs; *Salmonella dublin -* a cause of both a systemic and diarrhoeal disease in cattle, especially of new-born calves; *Escherichia coli* - a cause of food poisoning in humans; *Haemophilus influenzae* - a cause of meningitis; *Neisseria gonorrhoeae -* a cause of gomorrhoeae; *Yersinia enterocolitica -* the cause of a spectrum of diseases in humans ranging from gastroenteritis to fatal septicemic disease; *Brucella abortus -* a cause of abortion and infertility in cattle and a condition known as undulant fever in humans; or *Clostridium difficile -* a cause of pseudomembranous colitis.

Examples of antigens whose epitopes may be inserted are the pre-S1, pre-S2 and S antigens of HBV; the HAV surface antigens; the HCV surface antigens, core protein and NS3 protein; the HIV antigens gp120, gp160, gag, pol, Nef, Tat and Ref; the malaria antigens such as the circumpsorozoite proteins; the influenza antigens HA, NP and NA; the herpes virus antigens EBV gp340, EBV gp85, HSV gB, HSV gD, HSV gH and HSV early protein; the human papilloma virus antigens E4, E6 and E7; the cancer antigens carcinoembryonic antigen (CEA), P53, ras and myc; the pertactin antigen from *Bordetella pertussis;* and house dust mite allergen.

The invention is particularly suited to prophylactic or therapeutic vaccination against HBV since the carrier protein HBcAg is from HBV, and epitopes from the pre-S1, pre-S2 and S regions of HBV are particularly preferred. A pre-S1, pre-S2 or S insert is typically at least 6 amino acids in length, for example from 6 to 120 aa, 8 to 80 aa or 10 to 40 aa. The insert may include, for example, the residues at pre-S1 positions 1-9, 10-19, 20-29, 30-39, 40-49, 50-59, 60-69, 70-79, 80-89, 90-99, 100-109 or 110-119 or the residues at pre-S2 positions 120-129,130-139,140-149, 150-159,160-169 or 170-174. Particularly preferred fragments are those corresponding to pre-S1 residues 20-47 and pre-S2 residues 139-174. Pre-S1 residues 21-28 correspond to a human T-cell epitope. Also preferred are fragments corresponding to S residues 110-147 and 110-157 (counting the first residue of the S sequence as residue 1).

### Making the proteins of the invention

The proteins of the invention are generally made by recombinant DNA technology. The invention includes a nucleic acid molecule (e.g. DNA or RNA) encoding a protein of the invention, such as an expression vector.

The nucleic acid molecule may encode a protein in which one or more of the arginine repeats has been deleted and replaced with a restriction enzyme site unique to the nucleic acid molecule, such as an XbaI site. The nucleic acid molecule may also contain a unique restriction enzyme site in the sequence encoding the e1 loop and/or in the N-terminus. The unique restriction enzyme sites allow sequences encoding epitopes to be inserted into the nucleic acid molecule, for example in place of the deleted arginine repeat(s) or in the e1 loop.

A protein of the invention may be produced by culturing a host cell containing a nucleic molecule encoding the protein under conditions in which the protein is expressed, and recovering the protein. Suitable host cells include bacteria such as *E*. *coli,* yeast, mammalian cell lines and other eukaryotic cell lines, for example insect Sf9 cells.

The vectors constituting nucleic acid molecules according to the invention may be, for example, plasmid or virus vectors. They may contain an origin of replication, a promoter for the expression of the sequence encoding the protein, a regulator of the promoter such as an enhancer, a transcription stop signal, a translation start signal and/or a translation stop signal. The vectors may also contain one or more selectable marker genes, for example an ampicillin resistance gene in the case of a bacterial plasmid or a neomycin resistance gene for a mammalian vector. Vectors may be used *in vitro,* for example for the production of RNA or used to transform or transfect a host cell.

Promoters, enhancers and other expression regulation signals may be selected to be compatible with the host cell for which the expression vector is designed. For example, prokaryotic promoters may be used, in particular those such as the trc promoter suitable for use in *E*. *coli* strains (such as *E. coli* HB101). A promoter whose activity is induced in response to a change in the surrounding environment, such as anaerobic conditions, may be used. Preferably an *htrA* or *ni*rB promoter may be used. These promoters may be used in particular to express the protein in an attenuated bacterium, for example for use as a vaccine. When expression of the protein of the invention is carried out in mammalian cells *in vitro,* mammalian promoters may be used. Tissue-specific promoters, for example hepatocyte cell-specific promoters, may also be used. Viral promoters may also be used, for example the Moloney murine leukaemia virus long terminal repeat (MMLV LTR), the rous sarcoma virus (RSV) LTR promoter, the SV40 promoter, the human cytomegalovirus (CMV) IE promoter, herpes simplex virus promoters and adenovirus promoters. All these promoters are readily available in the art.

A protein according to the invention may be purified using conventional techniques for purifying proteins. The protein may, for example, be provided in purified, pure or isolated form. For use in a vaccine, the protein must generally be provided at a high level of purity, for example at a level at which it constitutes more than 80%, more than 90%, more than 95% or more than 98% of the protein in the preparation. However, it may be desirable to mix the protein with other proteins in the final vaccine formulation, for example other proteins comprising pre-S1, pre-S2 or S sequence ofHBV. The protein is preferably substantially free from nucleic acid (DNA and RNA).

### Vaccines

The primary use of the proteins of the invention is as therapeutic or prophylactic vaccines. The invention includes a vaccine composition comprising a protein of the invention or a particle of the invention and a pharmaceutically acceptable carrier or diluent.

The principle behind prophylactic vaccination is to induce an immune response in a host so as to generate an immunological memory in the host. This means that, when the host j-exposed to the virulent pathogen, it mounts an effective (protective) immune response, i.e an immune response which inactivates and/or kills the pathogen. The invention could form the basis of a prophylactic vaccine against a range of diseases, such as HBV, HAV, HCV, influenza, foot-and-mouth disease, polio, herpes, rabies, AIDS, dengue fever, yellow fever, malaria, tuberculosis, whooping cough, salmonellosis, typhoid, food poisoning, diarrhoea, meningitis and gonnorrhoeae. The epitopes in the protein of the invention are chosen so as to be appropriate for the disease against which the vaccine is intended to provide protection.

The principle behind therapeutic vaccination is to stimulate the immune system of the host to alleviate or eradicate a disease or condition. There are a number of diseases and conditions which may be susceptible to therapeutic vaccination, such as chronic viral diseases including chronic HBV and chronic HCV, cancer, and allergies such as asthma, atopy, eczema, rhinitis and food allergies.

Chronic viral diseases arise when the immune system of an infected host fails to eliminate the virus, allowing the virus to persist in the host for a long period of time. The invention may be used to induce the immune system of the chronically infected individual so as to eliminate the virus. For example, it is believed that patients with chronic hepatitis have an inadequate T-cell response, and that stimulation of an appropriate T-cell response can eliminate the virus. Thus, in order to treat viral hepatitis using the invention, T-cell epitopes may be inserted into the protein of the invention, such as T-cell epitopes from the pre-S1 and pre-S2 regions of HBV.

Similarly, in the case of cancer, it is believed that enhancement of the T-cell response to tumour antigens may help the immune system to destroy the tumour. It is believed that allergic diseases are caused at least in part by an unbalanced T-cell response in which an inflammatory Th2 responses dominates over an antagonistic Th1 response, and that allergies may therefore be treated by enhancing the Th1 response. This can be achieved according to the invention by using a protein which stimulates a Th1 response.

More than one protein according to the invention may be administered to a patient. Furthermore, a protein according to the invention may be used in combination with one or more other compositions. For example, in the treatment of chronic HBV a protein according to the invention may be used in combination with interferon gamma, Lamivudine™, or another immunotherapeutic agent such as Hepacare™ (formerly known as Hepagene™). The protein according to the invention and the other composition may be administered simultaneously or sequentially.

Suitable carriers and diluents for inclusion in pharmaceutical compositions of the invention are isotonic saline solutions, for example phosphate-buffered saline. The composition will normally include an adjuvant, such as aluminium hydroxide. The composition may be formulated for parenteral, intramuscular, intravenous, intranasal, subcutaneous or transdermal administration. The composition comprises the protein, particles or nucleic acid in a prophylactically or therapeutically effective amount. Typically, the protein or particles are administered at a dose of from 0.01 to 30 µg/kg body weight, preferably from 0.1 to 10 µg/kg, more preferably from 0.1 to 1 µg/kg body weight. The nucleic acid of the invention may be administered directly as a naked nucleic acid construct using techniques known in the art or using vectors known in the art. The amount of nucleic acid administered is typically in the range of from 1 µg to 10 mg, preferably from 100 µg to 1 mg. The vaccine may be given in a single dose schedule or a multiple dose schedule. The routes of administration and doses given above are intended only as a guide, and the route and dose may ultimately be at the discretion of the physician.

### Experimental Section

### Experiment 1

### 1. Materials and Methods

New plasmid constructs were generated by inverse PCR so that three or four C-terminal arginine repeat regions were deleted and a *Spe*I restriction site was introduced to allow insertion of replacement sequences coding for B and T cell epitopes (Fig.1).

The plasmid templates for the inverse PCR were *ptrc*/core and *ptrc*/core-S1 which encode respectively for non-hybrid hepatitis B core and hybrid hepatitis B core containing amino acids 20-47 of the pre-S1 sequence of hepatitis B surface protein inserted between amino acids 79 and 80 of the immunodominant e1 loop. Three oligonucleotide primers (Table 1 and Fig. 2) were used for the PCR reaction. These primers introduce a unique *SpeI* restriction site in the PCR fragments. The primers were also designed to generate new fragments that were truncated at residues 146 or 154 but maintained 7 residues of the C-terminus including the terminal cysteine at position 185 which is thought to be important for maintaining particle stability by formation of disulphide bonds (Fig. 1).

### 1.1 Construction of parental truncated plasmids

Using primers MCR371/370 or MGR369/370 (Table 1 and Figure 2), inverse PCR fragments are generated from plasmid templates of *ptrc*/*core* or ptrc/core-S1. This procedure removes 69 nucleotides (encoding for 23 amino acids (aa155-177)) and 93 nucleotides (encoding for 31 amino acids (146-177)) respectively. The PCR fragments sizes were confirmed by analysis on agarose gels and then digested with *SpeI* restriction endonuclease followed by purification on agarose gels and self-ligation to generate plasmids pTCR₁₄₆, pTCR₁₅₄ and pTCSR₁₄₆ and pTCSR₁₅₄. pTCR plasmids are derived from the ptrc/core template and pTCSR plasmids are derived from the *ptrc*/core-S1 templates. The 146 and 154 numbering denotes the amino acid number at the truncation point. The four parental truncated plasmids were used to transform *E. coli* HB101 cells and positive colonies were tested by diagnostic PCR using oligonucleotide primers MGR61/MGR168. Core protein expression was confirmed by immunoblotting of bacterial cell lysates using a mouse anti-core antibody.

### 1.2 Subcloning of replacement sequences into truncated parental plasmids

Three sequences have been subcloned into the 3' end of the truncated parental plasmids described in section 1.1. These include sequences encoding for amino acids 110-147 and 110-157 of the small hepatitis B surface protein, and aa20-55 of the S2 region of the middle hepatitis B surface protein (Figure 3).

For insertion of the 110-157 sequence (plus 2 amino acids resulting from the *Nhe*I restriction site) oligonucleotide primers MR245-247 (Table 1B) were used to generate a PCR fragment of 147 nucleotides using pMBdSRE/17 as template (Figure 3). This plasmid encodes for the small hepatitis B surface protein (adw subtype) for expression in mammalian cells using the mouse metallothionine promoter.

For insertion of the 110-147 sequence (plus 2 amino acids from the *Nhe*I site) oligonucleotide primers MGR247/264 (Table 1B) were used to generate a PCR fragment of 120 nucleotides using pMBdSRE/17 as template (Figure 3).

For insertion of the 20-55 sequence (plus 2 residues from the *NThe*I site) of pre-S2, oligonucleotide primers MGR243/249 (Table 1B) were used to generate a PCR fragment of 114 nucleotides using pMByS2R/8 as template (Figure 3). This plasmid encodes for the middle hepatitis B surface protein (ayw subtype) under control of the metallothionine promoter for mammalian cell expression.

The PCR fragments were digested with *Nhe*I restriction endonuclease and purified on agarose gels. The purified fragments were then ligated with *Spe*I digested, phosphatase treated parental plasmids (section 1.1). *E.coli* HB101 cells were then transformed with the resulting plasmids and positive colonies tested by diagnostic PCR using oligonucleotide primers MGR61/168, immunoblotting with antibodies specific for the insert and partial DNA sequencing of the inserts.

### 2. Results

### 2.1 Confirmation of inverse PCR fragment generation

Inverse PCR fragments for pTCR₁₄₆, pTCR₁₅₄, pTCSR₁₄₆ and pTCSR₁₅₄ were analysed by separation on 1% agarose gels (Figure 4). The PCR fragments were found to be of the appropriate size (approx. 5.2kb) and were confirmed to be correct by diagnostic PCR (not shown). Immunoblot analysis showed that the parental constructs and those containing the inserted sequences expressed the core protein that was reactive to an anti-core antibody (Figure 5). Further, confirmation of protein expression of the inserted sequences was shown by immunoblotting using anti-S (Figure 6) and anti-pre-S2 antibodies (Figure 7).

### Table 1. Oligonucleotide primers used for inverse and diagnostic PCR

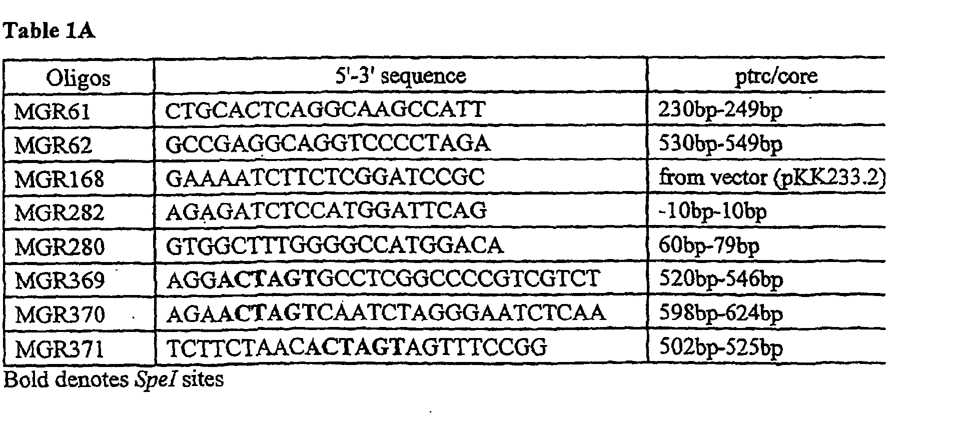

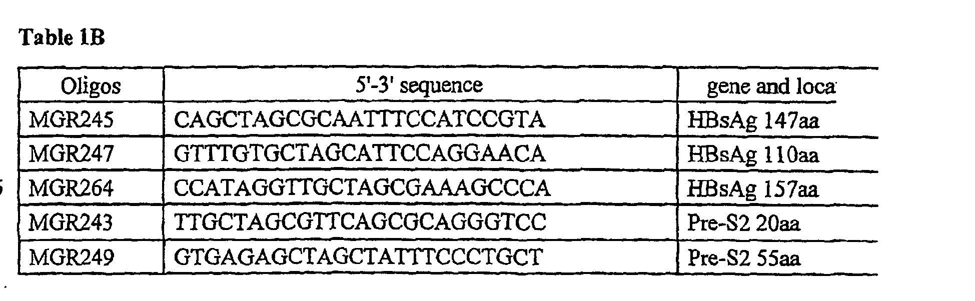

### Experiment 2

### Summary

Full-length and C-terminally truncated hepatitis B core antigen (HBc) derivatives, which carried long foreign amino acid insertions at position 144, were constructed. HBV preS1, preS2, and HIV-1 Gag fragments of 50-100 amino acids in length were used as such insertions, and the appropriate recombinant genes were expressed in *E.coli* cells. The appropriate chimeric HBc and HBcΔ derivatives were purified and examined antigenically and immunogenically. Subclass analysis of the induced anti-HBc immune response in mice showed that the Ig ratio of IgG1, IgG2a, and IgG2b antibodies was restored from the IgG1>IgG2a≥IgG2b pattern, which is typical for C-terminally truncated HBcΔ derivatives, to IgG2a≥IgG2b≥IgG1, which is typical for full-length HBc derivatives, after immunisation with C-terminally truncated HBcΔ derivatives which carried long C-terminal additions of 50-100 amino acids in length.

### Materials and Methods

### Bacterial Strains

*E.coli* strains RR1 (F, hsdS20 (r⁻ _{b}, m⁻_{b}), *recA*⁺*, ara*-14,*pro*A2, *lac*Y1 *, galK2, rps*L20 (Sm^{r}), *xyl-5, mtl-1, sup*E44*,* λ⁻ ), and K802 (*hsdR, gal, met, supE, mcrA, mcrB*) were used for selection and expression of chimeric genes, respectively.

### Animals

BALB/C (H-2^{d}) female mice were used approximately 7-10 weeks old, weight 20 mg. New Zealand white strain female rabbits were used for obtaining polyclonal antibodies.

### Construction of HBc Derivatives

*Vectors based on plasm ids pHBc3 and pHBc16-15.* Vectar pHBc3 was constructed by putting the HBc gene under the control of the tandem repeat of *E. coli trp* promoters. Vector pHBc 16-15 was constructed by insertion of an oligonucleotide linker carrying *Cla* I/Eco *RV* restriction sites into position 144 of the HBc gene.

*Construction of chimeric HBc derivatives.* The structure of the HBc and HBc△ derivatives is shown in Table 2. The recombinant genes were constructed by insertion of the appropriate HBV greS1, preS2, and HIV-1 gag fragments into the Cla I site ofthe pHBc16-15 vector, with or without in-frame junction to the C-terminal part of the HBc gene.

### Purification of Chimeric HBc Derivatives

*E.coli* cells were grown overnight on a rotary shaker at 37°C in 750 ml flasks containing 300 ml of M9 minimal medium supplemented with 1% casamino acids (Difco Laboratories, Sparks, USA) and 0.2% glucose. An optical density OD₅₄₀ of 2-5 was usually reached. Generally, cells were pelleted and lysed by 30 min incubation on ice in lysis buffer containing 50 mM Tris-HCl (pH 8.0), 5 mM EDTA, 50 µg/ml PMSF, 2 mg/ml lysozyme and then ultrasonicated 3 times for 15 s at 22 kHz. Lysates were then adjusted to 10 mM MgCl₂, and 20 µg/ml DNAase. After low speed centrifugation, proteins were precipitated from the supernatant with ammonium sulfate at 33% saturation for 1-2 h at 4°C. Pellets were resuspended in a standard PBS buffer containing 0.1% Triton X-100™, and 5 ml of the solutions were loaded on a Sepharose CL4B™ column (2.5 x 85 cm) and eluted with PBS buffer without Triton X-100. The presence of HBc polypeptides in fractions was tested by PAGE. Positive fractions were pooled and concentrated by ammonium sulfate precipitation at 33% saturation for 20 h at 4°C. Pellets were resuspended in PBS, or in Tris-saline buffer, 10 mM Tris-HCl (pH7.5), 150 mM NaCl, to a final concentration of about 5-20 mg/ml, dialyzed overnight against 2000 volumes of the same buffer and stored at -70°C or at -20°C in 50% glycerol.

### Polyacrylamide Gel Electrophoresis and Western Blotting

For PAGE analysis, bacteria were pelleted, suspended in SDS-gel electrophoresis sample buffer containing 2% SDS and 2% 2-mercaptoethanol and lysed by heating at 100°C for 5 min. The proteins were separated by Laemmli's polyacrylamide gel electrophoresis (PAGE) in a slab gel (150x150x0.75 mm) apparatus with a gradient 12-18% running gel and a 4% stacking gel. Western blotting was performed in general as described by Towbin et al (1979) in Proc. Natl. Acad. Sci. USA 76 4350-4354. Nitrocellulose sheets (0.2 µ, Millipore, Bedford, USA) were incubated with anti-HBc antibodies and anti-preS1 antibody in dilutions of 1:100 to 1:1000 overnight and then with anti-mouse IgG peroxidase conjugate (1:1000) for 1-2 h at room temperature. The reaction was developed with 3,3'-diaminobenadine. In parallel, gels were silver-stained according to Ohsawa and Ebata (1983) Anal. Biochem. 135 409-415.

### Immunisations

Mice (five per group) were immunised at day 0 intraperitoneally with 0.02 mg of chimeric particles in complete Freund's adjuvant (CFA, Difco) followed by two booster immunisations in Freund's incomplete adjuvant (IFA, Difco) given at days 10 (0.01 mg intraperitoneally) and 24 (0.01 mg intraperitoneally and 0.01 mg subcutaneously). Sera obtained on day 32 were analysed by ELISA for reactivity with HBc particles.

### ELISA

For the ELISA, recombinant HBc particles were coated onto 96-well microtiter plates by airdrying in a chemical hood overnight. Wells were blocked with 0.5% BSA in PBS for 1 h, incubated with serial dilutions of the various antibodies for 1 h at 37°C and processed with the appropriate second antibodies conjugated to horse radish peroxidase (Sigma) according to the protocols of the manufacturers. Plates were washed 5 times between incubations with 0.05% Tween-20™ in PBS, and 5 times with distilled water to remove Tween-20. Optical absorbances were measured at 492 nm in an automatic Immunoscan MS™ reader. The titres were calculated as the negative logarithms of the EC50 (effective concentration, 50%) seru dilution on the basis of sigmoidal dose-response curves. GraphPad Prism® version 3.. software was used in the mean titre calculations.

### Results

*Immunogenicity of Recombinant Proteins.* To measure the immunogenicity ofHBc carrier and inserted preS1, preS2, and Gag sequences, individual mice sera were repeatedly tested by direct ELISA using recombinant HBcAg and synthetic preS1, preS2, and HIV-1 p24 peptides on solid support. Immunisation with chimeric particles induced high levels of anti-HBc and relatively low levels of anti-insertion antibodies (not shown).

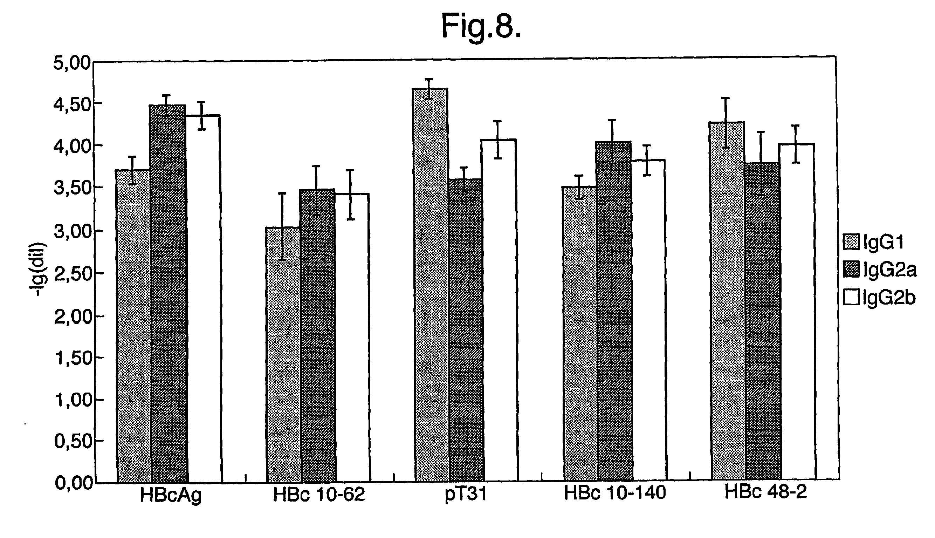

*Induction of Different Immunoglobulin Subclasses by Chimeric HBcΔ-preS1 (20-47) Particles* In order to average obtained immunisation data and to make them more informative for comparative subclass analysis of induced immunoglobulins, we calculated mean titres for each group of immunised animals as the negative logarithms of the EC50 (effective concentration, 50%) serum dilution on the basis of sigmoidal dose-response curves (GraphPad Prism® version 3.02). These data on the anti-HBc response of immunised mice, which allow direct comparison of averaged titres, are given in Fig. 8.

The data presented in Fig. 8 show that the wild type HBcAg induces anti-HBc response with the immunoglobulin subclass distribution IgG2a≥IgG2b≥IgG1, whilst the immune response to the C-terminally truncated HBc△ structure T31 presents the IgG1>IgG2b≥IgG2a subclass distribution pattern. The full-length HBc derivative 10-62, which carries a 50 aa long preS1 insertion, shows a subclass distribution analogous to that of the full-length HBc vector. Moreover, replacement of the C-terminus of the HBc molecule by a long foreign insertion (50 amino acids of the preS1 sequence) in the HBc derivative 10-140 makes the subclass distribution of the anti-HBc antibodies rather similar to that induced by the full-length HBc structure (Fig. 8). The HBcΔ derivative 48-2 with a 100 aa long insertion of HIV-1 Gag occupies an intermediate position in this sense between wild type HBcAg and C-terminally truncated HBcΔ T31 structures.

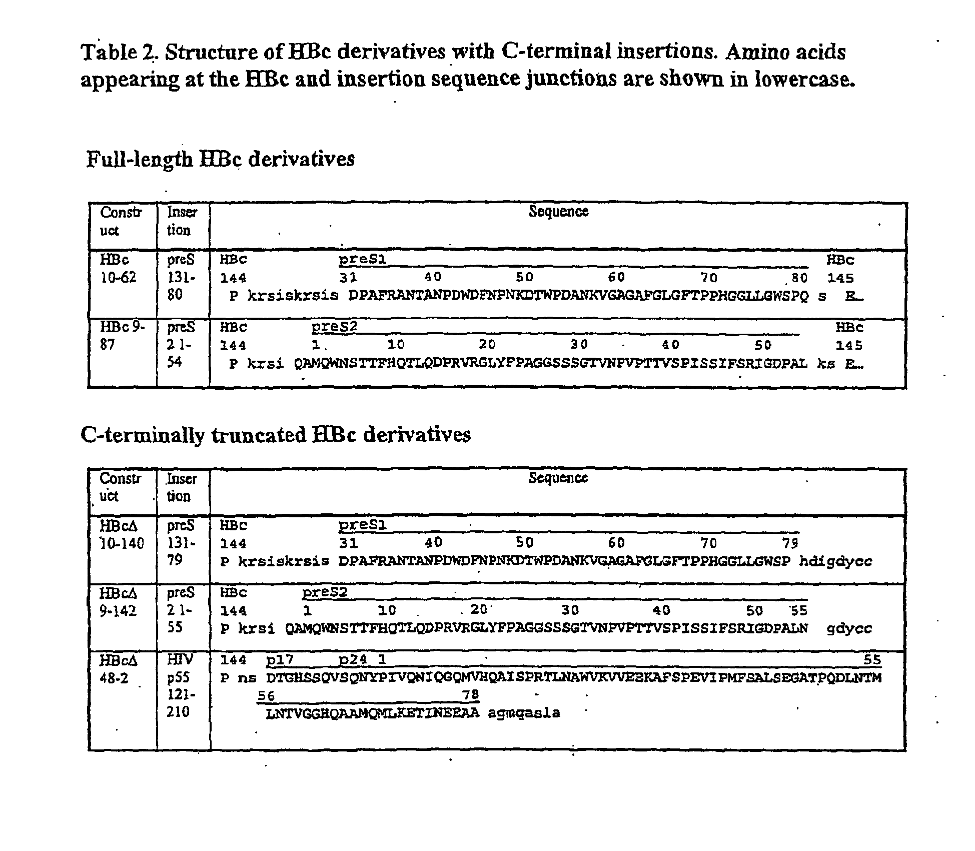

## Claims

1. A vaccine composition comprising a protein comprising hepatitis B core antigen (HBcAg) wherein one or more of the four arginine repeats is absent and a C-terminal cysteine residue is present, and a pharmaceutically acceptable carrier or diluent.

2. A composition according to claim 1 wherein a first epitope from a protein other than HBcAg is present in place of the absent arginine repeat(s).

3. A composition according to claim 1 or 2 wherein the first arginine repeat is present and the second to fourth arginine repeats are absent.

4. A composition according to any one of the preceding claims wherein a sequence lying between residues 145 and 182 of HBcAg is absent.

5. A composition according to any one of the preceding claims wherein a sequence lying between residues 150 and 177 of HBcAg is absent.

6. A composition according to any one of the preceding claims which comprises a second epitope from a protein other than HBcAg, the second epitope being in the e1 loop.

7. A composition according to claim 6 wherein the second epitope is a B-cell epitope.

8. A composition according to any one of claims 2 to 7 wherein the first epitope is a T-cell epitope.

9. A composition according to claim 8 wherein the first epitope is a T-helper cell epitope and the second epitope is a B-cell epitope.

10. A composition according to claim 6 which comprises said first and second epitopes wherein the epitopes are the same.

11. A composition according to any one of claims 2 to 10 wherein the first and/or the second epitope is from hepatitis B virus (HBV).

12. A composition according to claim 11 wherein the first and/or the second epitope is from the pre-S1, pre-S2 or S region of HBV.

13. A composition according to claim 1 comprising the following elements linked in an N-terminal to C-terminal direction:
(i) and N-terminal part of HBcAg which mediates the formation of particles, and
(ii) a C-terminal part of HBcAg comprising the C-terminal cysteine;
wherein at least a part of the sequence ofHbcAg from between said N-terminal part and said C-terminal part comprising one or more of the arginine repeats is absent.

14. A composition according to claim 1 comprising the following elements linked in an N- to C- terminal direction:
(i) an N-terminal part of HBcAg which mediates the formation of particles,
(ii) an epitope from a protein other than HBcAg, and
(iii) a C-terminal part of HBcAg comprising the C-terminal cysteine;
wherein at least a part of the sequence of HBcAg between said N-terminal part and said C-terminal part comprising one or more of the arginine repeats is absent and is replaced by said epitope.

15. A composition according to claim 1 comprising the following elements linked in an N- to C- terminal direction:
(i) an N-terminal part of the HBcAg sequence comprising residues 1 to 67,
(ii) an epitope from a protein other than HBcAg,
(iii) a second part of the HBcAg sequence comprising residues 91 to 144, and
(iv) a third part of the HBcAg sequence comprising the C-terminal cysteine;
wherein at least a part of the sequence of HBcAg from between residues 145 and the C-terminal cysteine comprising one or more of the arginine repeats is absent.

16. A composition according to claim 1 comprising the following elements linked in an N- to C- terminal direction:
(i) an N-terminal part of the HBcAg sequence comprising residues 1 to 67,
(ii) an epitope from a protein other than HBcAg,
(iii) a second part of the HBcAg sequence comprising residues 91 to 144,
(iv) a further epitope from a protein other than HbcAg,
(v) a third part of the HBcAg sequence comprising the C-terminal cysteine;
wherein at least a part of the sequence of HBcAg from between residues 145 and the C-terminal cysteine comprising one or more of the arginine repeats is absent.

17. A vaccine composition comprising a particle comprising multiple copies of a protein as defined in any one of the preceding claims, and a pharmaceutically acceptable carrier or diluent.

18. A protein as defined in any one of claims 1 to 16, or a particle as defined in claim 17 for use in a method of prophylactic or therapeutic vaccination of the human or animal body.

19. A protein or particle according to claim 18 for use in a method of prophylactic or therapeutic vaccination of the human or animal body against HBV.

20. Use of a protein as defined in any one of claims 1 to 16, or a particle as defined in claim 17 for the manufacture of a medicament for prophylactic or therapeutic vaccination of the human or animal body against HBV.

## Revendications

1. Composition vaccinale comprenant une protéine comprenant l'antigène core de l'hépatite B (HBcAg) dans laquelle une ou plusieurs des quatre répétitions d'arginines est (sont) absente(s) et un résidu cystéine C-terminal est présent, et un véhicule ou diluant pharmaceutiquement acceptable.

2. Composition selon la revendication 1 dans laquelle un premier épitope d'une protéine autre que HBcAg est présent à la place de la (des) répétition(s) d'arginines absente(s).

3. Composition selon la revendication 1 ou 2 dans laquelle la première répétition arginines est présente et la seconde jusqu'à la quatrième répétitions d'arginines sont absentes.

4. Composition selon l'une quelconque des revendications précédentes dans laquelle une séquence située entre les résidus 145 et 182 de HBcAg est absente.

5. Composition selon l'une quelconque des revendications précédentes dans laquelle une séquence située entre les résidus 150 et 177 de HBcAg est absente.

6. Composition selon l'une quelconque des revendications précédentes qui comprend un second épitope d'une protéine autre que HBcAg, le second épitope étant dans la boucle e1.

7. Composition selon la revendication 6, dans laquelle le second épitope est un épitope de lymphocyte B.

8. Composition selon l'une quelconque des revendications 2 à 7 dans laquelle le premier épitope est un épitope de lymphocyte T.

9. Composition selon la revendication 8 dans laquelle le premier épitope est un épitope de lymphocyte T auxiliaire et le second épitope est un épitope de lymphocyte B.

10. Composition selon la revendication 6 qui comprend lesdits premier et second épitopes dans laquelle les épitopes sont identiques.

11. Composition selon l'une quelconque des revendications 2 à 10 dans laquelle le premier et/ou le second épitope provient du virus de l'hépatite B (HBV).

12. Composition selon la composition 11 dans laquelle le premier et/ou second épitope provient de la région pré-S1, pré-S2 ou S de HBV.

13. Composition selon la revendication 1 comprenant les éléments suivants liés dans une direction N-terminale vers C-terminale :
(i) une partie N-terminale de HBcAg qui médie la formation de particules, et
(ii) une partie C-terminale de HBcAg comprenant la cystéine C-terminale ; dans laquelle au moins une partie de la séquence de HBcAg d'entre ladite partie N-terminale et ladite partie C-terminale comprenant une ou plusieurs des répétitions d'arginines est absente.

14. Composition selon la revendication 1 comprenant les éléments suivants liés dans la direction N- vers C-terminale :
(i) une partie N-terminale de HBcAg qui médie la formation de particules, et
(ii) un épitope d'une protéine autre que HBcAg, et
(iii)une partie C-terminale de HBcAg comprenant la cystéine C-terminale ; dans laquelle au moins une partie de la séquence de HBcAg entre ladite partie N-terminale et ladite partie C-terminale comprenant une ou plusieurs des répétitions d'arginines est absente et remplacée par ledit épitope.

15. Composition selon la revendication 1 comprenant les éléments suivants liés dans la direction N- vers C-terminale :
(i) une partie N-terminale de la séquence de HBcAg comprenant les résidus 1 à 67,
(ii) un épitope d'une protéine autre que HBcAg,
(iii)une seconde partie de la séquence de HBcAg comprenant les résidus 91 à 144, et
(iv) une troisième partie de la séquence de HBcAg comprenant la cystéine C-terminale ;
dans laquelle au moins une partie de la séquence de HBcAg d'entre les résidus 145 et la cystéine C-terminale comprenant une ou plusieurs des répétitions d'arginines est absente.

16. Composition selon la revendication 1 comprenant les éléments suivants liés dans la direction N- vers C-terminale :
(j) une partie N-terminale de la séquence de HBcAg comprenant les résidus 1 à 67,
(ii) un épitope d'une protéine autre que HBcAg,
(iii)une seconde partie de la séquence de HBcAg comprenant les résidus 91 à 144, et
(iv) un autre épitope d'une protéine autre que HBcAg,
(v) une troisième partie de la séquence de HBcAg comprenant la cystéine C-terminale ;
dans laquelle au moins une partie de la séquence de HBcAg d'entre les résidus 145 et la cystéine C-terminale comprenant une ou plusieurs des répétitions d'arginines est absente.

17. Composition vaccinale comprenant une particule comprenant des copies multiples d'une protéine comme défini dans l'une quelconque des revendications précédentes, et un véhicule ou diluant pharmaceutiquement acceptable.

18. Protéine telle que définie dans l'une quelconque des revendications 1 à 16, ou une particule telle que définie dans la revendication 17 utilisable dans une méthode de vaccination prophylactique ou thérapeutique du corps humain ou animal.

19. Protéine ou particule selon la revendication 18 utilisable dans une méthode de vaccination prophylactique ou thérapeutique du corps humain ou animal contre HBV.

20. Utilisation d'une protéine telle que définie dans l'une quelconque des revendications 1 à 16, ou une particule telle que définie dans la revendication 17 pour la fabrication d'un médicament pour la vaccination prophylactique ou thérapeutique du corps humain ou animal contre HBV.

## Patentansprüche

1. Impfstoffzusammensetzung, umfassend ein Protein, das ein Hepatitis-B-Core-Antigen (HbcAg) umfasst, worin ein oder mehrere der vier Arginin-Wiederholungen ("Repeats") abwesend sind und ein C-terminaler Cysteinrest vorhanden ist, und einen pharmazeutisch annehmbaren Träger oder Verdünnungsmittel.

2. Zusammensetzung nach Anspruch 1, worin ein erstes Epitop von einem Protein, das ein anderes als HBcAg ist, anstelle des/der abwesenden Arginin-Wiederholung(en) vorhanden ist.

3. Zusammensetzung nach Anspruch 1 oder 2, worin die erste Arginin-Wiederholung vorhanden ist und die zweiten bis vierten Arginin-Wiederholungen abwesend sind.

4. Zusammensetzung nach einem der vorangehenden Ansprüche, worin eine zwischen Resten 145 und 182 von HBcAg liegende Sequenz abwesend ist.

5. Zusammensetzung nach einem der vorangehenden Ansprüche, worin eine zwischen Resten 150 und 177 von HBcAg liegende Sequenz abwesend ist.

6. Zusammensetzung nach einem der vorangehenden Ansprüche, welche ein zweites Epitop von einem Protein, das ein anderes als HBcAg ist, umfasst, welches zweite Epitop in der E1-Schieife liegt.

7. Zusammensetzung nach Anspruch 6, worin das zweite Epitop ein B-Zell-Epitop ist.

8. Zusammensetzung nach einem der Ansprüche 2 bis 7, worin das erste Epitop ein T-Zell-Epitop ist.

9. Zusammensetzung nach Anspruch 8, wobei das erste Epitop ein T-Helferzell-Epitop ist und das zweite Epitop ein B-Zell-Epifop ist.

10. Zusammensetzung nach Anspruch 6, welche die ersten und zweiten Epitope umfasst, wobei die Epitope dieselben sind.

11. Zusammensetzung nach einem der Ansprüche 2 bis 10, wobei das erste und/oder das zweite Epitop von Hepatitiv-B-Virus (HBV) stammt.

12. Zusammensetzung nach Anspruch 11, wobei das erste und/oder das zweite Epitop aus der pre-S1-, pre-S2- oder S-Region von HBV stammt.

13. Zusammensetzung nach Anspruch 1, umfassend die folgenden, in einer N-terminalen nach C-terminalen Richtung verknüpften Elemente:
(i) einen N-terminalen Teil von HBcAg. welcher die Bildung von Partikeln vermittelt, und
(ii) einen C.terminalen Teil von HBcAg, umfassend das C-terminale Cystein; worin zumindest ein Teil der Sequenz von HBcAg aus zwischen dem N-terminalen Teil und dem C-terminalen Teil, umfassend ein oder mehrere der Arginin-Wiederholungen, abwesend ist.

14. Zusammensetzung nach Anspruch 1, umfassend die folgenden, in einer N- nach C-terminalen Richtung verknüpften Elemente:
(i) einen N-terminalen Teil von HBcAg, welcher die Bildung von Partikeln vermittelt,
(ii) ein Epitop von einem Protein, das ein anderes als HbcAg ist; und
(iii) einen C.terminalen Teil von HBcAg, umfassend das C-terminale Cystein; worin zumindest ein Teil der Sequenz von HBcAg zwischen dem N-terminalen Teil und dem C-terminalen Teil, umfassend ein oder mehrere der Arginin-Wiederholungen, abwesend ist und durch das Epitop ersetzt ist.

15. Zusammensetzung nach Anspruch 1, umfassend die folgenden, in einer N- nach C-terminalen Richtung verknüpften Elemente:
(i) einen N-terminalen Teil der HBcAg-Sequenz, umfassend Reste 1 bis 67,
(ii) ein Epitop von einem Protein, das ein anderes als HbcAg ist,
(iii) einen zweiten Teil der HBcAg-Sequenz, umfassend Reste 91 bis 144, und
(iv) einen dritten Teil der HBcAg-Sequenz, umfassend das C-terminale Cystein; worin zumindest ein Teil der Sequenz von HBcAg aus zwischen Resten 145 und dem C-terminalen Cystein, umfassend ein oder mehrere der Arginin-Wiederholungen, abwesend ist.

16. Zusammensetzung nach Anspruch 1, umfassend die folgenden, in einer N- nach C-terminalen Richtung verknüpften Elemente:
(i) einen N-terminalen Teil der HBcAg-Sequenz, umfassend Reste 1 bis 67,
(ii) ein Epitop von einem Protein, das ein anderes als HBcAg ist,
(iii) einen zweiten Teil der HBcAg-Sequenz, umfassend Reste 91 bis 144,
(iv) ein weiteres Epitop von einem Protein, das ein anderes als HbcAg ist,
(v) einen dritten Teil der HBcAg-Sequenz, umfassend das C-terminale Cystein; worin zumindest ein Teil der Sequenz von HBcAg aus zwischen Resten 145 und dem C-terminalen Cystein, umfassend ein oder mehrere der Arginin-Wiederholungen, abwesend ist.

17. Impfstoffzusammensetzung, umfassend einen Partikel, der mehrfache Kopien eines Proteins, wie in einem der vorangehenden Ansprüche definiert, und einen pharmazeutisch annehmbaren Träger oder Verdünnungsmittel umfasst.

18. Protein nach einem der Ansprüche 1 bis 16, oder ein Partikei nach Anspruch 17, zur Verwendung bei einer Methode zur prophylaktischen oder therapeutischen Imfpung des menschlichen oder tierischen Körpers.

19. Protein oder Partikel nach Anspruch 18 zur Verwendung bei einer Methode zur prophylaktischen oder therapeutischen Impfung des menschlichen oder tierischen Körpers gegen-HBV.

20. Verwendung eines Proteins nach einem der Ansprüche 1 bis 16, oder eines Partikels nach Anspruch 17. zur Herstellung eines Medikaments für die prophylaktische oder therapeutische Impfung des menschlichen oder tierischen Körpers gegen HBV.


### SEQUENCE LISTING

<110> MEDEVA EUROPE LTD
<120> MODIFICATION OF HEPATITIS B CORE ANTIGEN
<130> P78451A
<160> 2
<170> PatentIn Ver. 2.1
<210> 1
   <211> 639
   <212> DNA
   <213> Hepatitis B virus
<220>
   <221> CDS
   <222> (1)..(639)
<400> 1
   atg caa ctt ttt cac ctc tgc cta atc atc tct tgt tca tgt cct act    48
   Met Gln Leu Phe His Leu Cys Leu Ile Ile Ser Cys Ser Cys Pro Thr
     1               5                  10                  15
   gtt caa gcc tcc aag ctg tgc ctt ggg tgg ctt tgg ggc atg gac atc    96
   Val Gln Ala Ser Lys Leu Cys Leu Gly Trp Leu Trp Gly Met Asp Ile
                20                  25                  30
   gac cct tat aaa gaa ttt gga gct act gtg gag tta ctc tcg ttt ttg    144
   Asp Pro Tyr Lys Glu Phe Gly Ala Thr Val Glu Leu Leu Ser Phe Leu
            35                  40                  45
   cct tct gac ttc ttt cct tca gta cga gat ctt cta gat acc gcc tca    192
   Pro Ser Asp Phe Phe Pro Ser Val Arg Asp Leu Leu Asp Thr Ala Ser
        50                  55                  60
   gct ctg tat cgg gaa gcc tta gag tct cct gag cat tgt tca cct cac    240
   Ala Leu Tyr Arg Glu Ala Leu Glu Ser Pro Glu His Cys Ser Pro His
    65                  70                  75                  80
   cat act gca ctc agg caa gca att ctt tgc tgg ggg gaa cta atg act    288
   His Thr Ala Leu Arg Gln Ala Ile Leu Cys Trp Gly Glu Leu Met Thr
                   85                  90                  95
   cta gct acc tgg gtg ggt gtt aat ttg gaa gat cca gcg tct aga gac    336
   Leu Ala Thr Trp Val Gly Val Asn Leu Glu Asp Pro Ala Ser Arg Asp
               100                 105                 110
   cta gta gtc agt tat gtc aac act aat atg ggc cta aag ttc agg caa    384
   Leu Val Val Ser Tyr Val Asn Thr Asn Met Gly Leu Lys Phe Arg Gln
           115                 120                 125
   ctc ttg tgg ttt cac att tct tgt ctc act ttt gga aga gaa aca gtt    432
   Leu Leu Trp Phe His Ile Ser Cys Leu Thr Phe Gly Arg Glu Thr Val
       130                 135                 140
   ata gag tat ttg gtg tct ttc gga gtg tgg att cgc act cct cca gct    480
   Ile Glu Tyr Leu Val Ser Phe Gly Val Trp Ile Arg Thr Pro Pro Ala
   145                 150                 155                 160
   tat aga cca cca aat gcc cct atc cta tca aca ctt ccg gag act act    528
   Tyr Arg Pro Pro Asn Ala Pro Ile Leu Ser Thr Leu Pro Glu Thr Thr
                   165                 170                 175
   gtt gtt aga cga cga ggc agg tcc cct aga aga aga act ccc tcg cct    576
   Val Val Arg Arg Arg Gly Arg Ser Pro Arg Arg Arg Thr Pro Ser Pro
               180                 185                 190
   cgc aga cga agg tct caa tcg ccg cgt cgc aga aga tct caa tct cgg    624
   Arg Arg Arg Arg Ser Gln Ser Pro Arg Arg Arg Arg Ser Gln Ser Arg
           195                 200                 205
   gaa tct caa tgt tag                                                639
   Glu Ser Gln Cys
       210
<210> 2
   <211> 212
   <212> PRT
   <213> Hepatitis B virus
<400> 2
   Met Gln Leu Phe His Leu Cys Leu Ile Ile Ser Cys Ser Cys Pro Thr
    1               5                  10                  15
   Val Gln Ala Ser Lys Leu Cys Leu Gly Trp Leu Trp Gly Met Asp Ile
               20                  25                  30
   Asp Pro Tyr Lys Glu Phe Gly Ala Thr Val Glu Leu Leu Ser Phe Leu
   35 40 45
   Pro Ser Asp Phe Phe Pro Ser Val Arg Asp Leu Leu Asp Thr Ala Ser
   50 55 60
   Ala Leu Tyr Arg Glu Ala Leu Glu Ser Pro Glu His Cys Ser Pro His
   65 70 75 80
   His Thr Ala Leu Arg Gln Ala Ile Leu Cys Trp Gly Glu Leu Met Thr
   85 90 95
   Leu Ala Thr Trp Val Gly Val Asn Leu Glu Asp Pro Ala Ser Arg Asp
   100 105 110
   Leu Val Val Ser Tyr Val Asn Thr Asn Met Gly Leu Lys Phe Arg Gln
   115 120 125
   Leu Leu Trp Phe His Ile Ser Cys Leu Thr Phe Gly Arg Glu Thr Val
   130 135 140
   Ile Glu Tyr Leu Val Ser Phe Gly Val Trp Ile Arg Thr Pro Pro Ala
   145 150 155 160
   Tyr Arg Pro Pro Asn Ala Pro Ile Leu Ser Thr Leu Pro Glu Thr Thr
   165 170 175
   Val Val Arg Arg Arg Gly Arg Ser Pro Arg Arg Arg Thr Pro Ser Pro
   180 185 190
   Arg Arg Arg Arg Ser Gln Ser Pro Arg Arg Arg Arg Ser Gln Ser Arg
   195 200 205
   Glu Ser Gln Cys
   210